# EUROPEAN PATENT APPLICATION

(11) **EP 2 491 971 A1**
(43) Date of publication of application: **29.08.2012**
(21) Application number: 11155651.0
(22) Date of filing: 23.02.2011
(51) Int. Cl.: A61M 5/34

(54) **Needle adapter**

(71) Applicant: Sanofi-Aventis Deutschland GmbH, 65929 Frankfurt (DE)
(72) Inventor: The designation of the inventor has not yet been filed

(57) **Abstract**

The invention relates to a needle adapter (2) for coupling a needle (3) to an injection device (4). The needle adapter (2) comprises a proximal portion for receiving a distal end of the injection device (4), and a distal portion having an opening for receiving the needle (3).

## Description

### Technical Field

The invention relates to a needle adapter for coupling a needle to an injection device.

### Background of the Invention

Conventionally, single-use needle assemblies are provided for use with injection devices, e.g., insulin pens. The needle assemblies generally include a needle assembly integrally formed with a double-sided needle and include a means (e.g., threads, friction) for detachably coupling to a distal end of the injection device. After use, the needle assembly is removed from the injection device and discarded.

Many needle assemblies are relatively large and become difficult to package and carry. Further, it would be more cost-effective to use replaceable needles rather than larger, more expensive needle assemblies. Thus, there exists a need for a more efficient needle assembly configuration.

### Summary of the Invention

It is an object of the present invention to provide an improved needle adapter for coupling a needle and an injection device. In an exemplary embodiment, the needle adapter is sized and shaped to be coupled to a variety of injection devices, e.g., insulin pens. For example, the needle adapter may be sized and shaped like a Type A needle assembly. As understood by those of skill in the art, such a needle adapter may allow legacy injection devices to support the use of single-use needles, which may be more economical, more efficient and easier to use than single-use needle assemblies.

The exemplary embodiments of the present invention describe a needle adapter for coupling a needle to an injection device. The needle adapter may comprise a proximal portion for receiving a distal end of the injection device, and a distal portion having an opening for receiving the needle.

The proximal portion of the needle adapter may have a screw thread on an inner surface, e.g., for engaging a corresponding screw thread on the injection device. The needle adapter may include a longitudinal needle guide formed along a central axis of the needle adapter for receiving the needle. A retaining element may be formed along a portion of the needle guide for receiving a retaining member formed on the needle. A diameter of the retaining element may be larger than a diameter of the needle guide. A proximal end of the retaining element may prevent proximal displacement of the retaining member in the needle guide beyond the retaining element. A tactile feedback may be provided when the retaining member is received within the retaining element.

The retaining member may be conically shaped, narrowing in a proximal direction, and the opening in the distal portion of the needle adapter may be conically shaped, narrowing in the proximal direction. At least one radial slot may be formed in the distal portion, wherein at least one end of each of the at least one radial slots terminates at the opening. The at least one radial slot may form at least one latch, which may prevent distal displacement of the retaining member in the needle guide beyond the retaining element.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, do not limit the present invention, and wherein:
- Figure 1: shows a perspective view of an exemplary embodiment of a needle adapter coupled to an injection device according to the present invention,
- Figure 2: shows a sectional view of an exemplary embodiment of a needle adapter coupled to an injection device according to the present invention;
- Figure 3: shows an exploded sectional view of an exemplary embodiment of a needle adapter and a corresponding needle according to the present invention;
- Figure 4: shows an exemplary embodiment of a needle adapter and a corresponding needle according to the present invention; and
- Figure 5: shows an exemplary embodiment of a removal device for a needle according to the present invention.

Corresponding parts are marked with the same reference symbols in all figures.

### Detailed Description of Preferred Embodiments

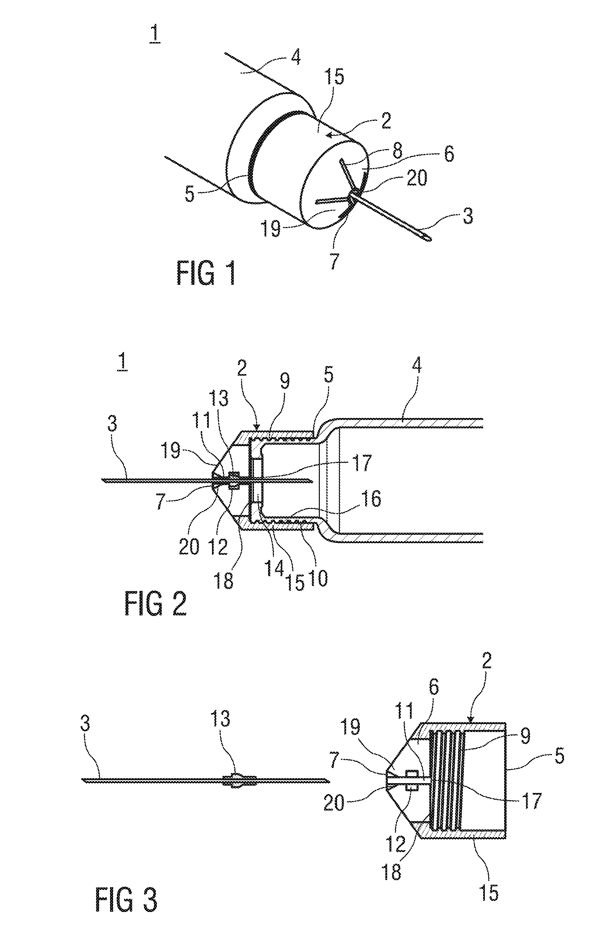

Figures 1 and 2 show a perspective view and a sectional view, respectively, of an exemplary embodiment of an injection device assembly 1 with a needle adapter 2 coupling a needle 3 (e.g., a double-sided/tipped needle) to an injection device 4. The injection device 4 may be designed as, for example, a cartridge holder for a cartridge containing a medicament.

Figure 3 shows an exploded sectional view of the needle adapter 2 and the needle 3 shown in Figures 1 and 2.

In an exemplary embodiment, the needle adapter 2 may have a proximal portion 5 that is shaped to removably engage a distal end of the injection device 4, cartridge holder or adapter attached thereto. For example, the proximal portion 5 may have the shape of a hollow cylinder with an open proximal end which is shaped and sized to receive the distal end of the injection device 4. A distal end 6 of the needle adapter 2 may include an opening 7 suited for receiving the needle 3.

In an exemplary embodiment, the distal end 6 may have a conical shape tapering from a proximal cylindrical portion 15 of the needle adapter 2 to the opening 7 in the distal end 6. Furthermore, the distal end 6 may comprise one or more slots 8 extending radially from the opening 7. The slots 8 may form one or more latches 9.

In an exemplary embodiment, an inner diameter of a proximal portion 15 of the needle adapter 2 may correspond to an outer diameter of a distal end 16 of the injection device 4. The needle adapter 2 may comprise a screw thread formed as an internal thread 9 on an inner surface of the cylindrical portion 15 of the needle adapter 2. The injection device 4 may comprise a corresponding external thread 10 formed on an outer surface of the distal end 16. The internal thread 9 and the external thread 10 may provide a mechanism for removably mounting the needle adapter 2 on the injection device 4. Those of skill in the art will understand that other mounting mechanisms such as, for example, snap-fit, bayonet-fit, frictional fits, etc., may be used for mounting the needle adapter 2 on the injection device 4.

The needle adapter 2 further comprises a needle guide 11 for receiving the needle 3. In an exemplary embodiment, the needle guide 11 may be a longitudinal channel formed along a central axis of the needle adapter 2 terminating distally at the opening 7 and proximally at a proximal opening 17 formed in a proximal surface 18 of the needle adapter 2 which abuts a distal surface of the injection device 4.

Formed along a portion of the needle guide 11 may be a retaining element 12 for engaging a retainer member 13 formed (e.g., overmolded) on the needle 3. In an exemplary embodiment, the retaining element 12 may be an annular cut-out formed along a portion of the needle guide 11 which has a larger diameter than the needle guide 11. The diameter or space occupied by the retaining element 12 may correspond to a size and shape of the retainer member 13 on the needle 3. After the needle adapter 2 is coupled to the injection device 4, the needle 3 can be coupled to the needle adapter 2 by inserting the needle 3 into the needle guide 11. There may be a tactile feedback provided in that resistance will be encountered when moving the needle 3 proximally through the needle guide 11 until the retaining member 13 reaches the retaining element 12, at which point a user will feel a release of the resistance. Preferably the retaining element 12 has a proximal portion (e.g., surface/wall) which prevents further proximal movement of the needle 3 in the needle guide 11. When the needle 3 has been inserted into the needle adapter 2 such that the retaining member 13 is located in the retaining element 12, a proximal tip of the needle 3 will have been inserted into a medicament cartridge in the injection device 4.

The opening 7 at the distal end of the needle adapter 2 may be configured to facilitate insertion (and/or removal) of the needle 3. In an exemplary embodiment, the opening 7 may comprise a conically shaped inner surface 20 narrowing in the proximal direction. In this exemplary embodiment, the retaining member 13 on the needle 2 may be conically (or frusto-conically) shaped, and narrowing in a proximal direction, to engage the opening 7. The corresponding shapes and sizes of the inner surface 20 of the opening 7 and the retaining member 13 may allow the user to properly align the needle 3 with needle guide 11.

When the needle 3 is inserted into the needle guide 11, an outer surface of the retaining member 13 may engage and inner surface 20 of the opening 7, and as the needle 3 is advanced proximally, the latches 19 may bend proximally. When a distal end of the retaining member 13 passes the latches 19, the retaining member 13 will be secured in the retaining element 12. The latch 19 may be formed to prevent distal displacement of the needle 3 when the retaining member 13 has been secured in the retaining element 12, and a proximal surface of the retaining element 12 may be formed to prevent proximal displacement of the needle 3.

After the retaining member 13 has been secured in the retaining element 12, there should be a sufficient length of the needle 3 proximal to the retaining member 13 that would allow a proximal tip of the needle 3 to enter the medicament cartridge.

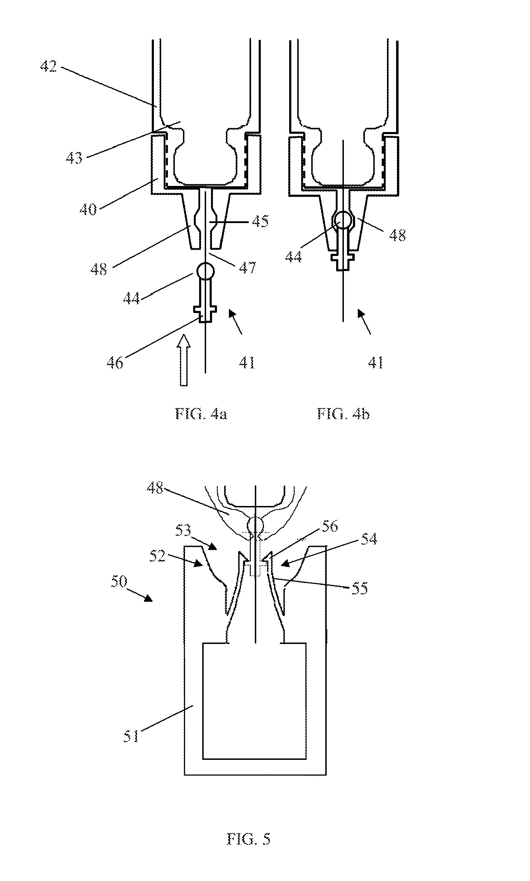

Figures 4a and 4b show another exemplary embodiment of a needle adapter 40 coupling a needle 41 to an injection device 42. The needle 41 may be a double-sided needle including proximal and distal tips. The proximal tip of the needle may be inserted into a cartridge 43 in the injection device 42 which contains a medicament, and the distal tip may be used to pierce a patient's skin for delivery of the medicament.

The needle 41 may include a retaining member 44 sized and shaped to fit within a retaining element 45 formed in the needle adapter 40. The needle 41 may further include a removal member 46 which may be used to remove the needle 41 from the needle adapter 40. The retaining member 44 and/or the removal member 46 may be formed as one or more overmolded portions on the needle 41. In an exemplary embodiment, the retaining member 44 may be formed as a sphere and the removal member 46 may be formed as a disc or inverted cross.

To couple the needle 41 to the needle adapter 40, the needle 41 may be inserted into an opening 47 formed in a distal end of the needle adapter 40. Preferably a diameter or cross-section of the retaining member 44 is larger than a diameter or cross-section of the opening 47. However, the distal end of the needle adapter 40 may comprise a plurality of arms 48 which may be formed by slots (not shown) created in the distal end. That is, the distal end of the needle adapter 40 may be a solid, conical piece, and one or more slots may be formed in the conical piece to create the arms 48. When the retaining member 44 is pushed proximally into the opening 47, the arms 48 may move from a first position radially outward to a second position to allow the retaining member 44 to pass through to the retaining element 45. When the retaining member 44 is secured within the retaining element 45, the arms 48 may return to the first position, ensuring that the needle 41 maintains its position relative to the needle adapter 40. When the retaining member 44 is secured within the retaining element 45, the proximal tip of the needle is located within the cartridge 43, and the injection device 42 may be ready to deliver an injection.

Figure 5 shows an exemplary embodiment of the needle adapter 40 and a needle removal device 50. In an exemplary embodiment, the removal device 50 may be a container 51 with a removal mechanism 52 formed integrally with the container 51, such that when the needle 41 is removed from the needle adapter 40, the needle 41 is permanently stored in the container 51.

In an exemplary embodiment, the removal mechanism 52 may include an opening 53 which is sized and shaped to receive a distal end of the injection device 42, the needle 41 and the needle adapter 40. A depth of the opening 53 may be varied. For example, the opening 53 may be relatively shallow, sized to receive the needle 41 and needle adapter 40. Alternatively, the opening 53 may be relatively deep, sized to receive the needle 41, the needle adapter 40 and a portion or all of the length of the injection device 42.

A removal element 54 may be formed at a distal end of the opening 53 and may be similar to the distal end of the needle adapter 40. For example, the removal member 54 may comprise a plurality of arms 55 which may be formed by slots (not shown) created in a proximal portion (e.g., a solid, conical piece) extending proximally from a distal end of the opening 53. The arms 55 may include latches 56 formed their proximal ends, and a receiving space 56 sized and shaped to receive the removal member 46 may be formed between the proximal ends of the arms 55.

When the injection device 42 is advanced distally into the opening 53, the removal member 46 causes the arms 55 of the removal element 54 to move radially outward. As the removal member 46 moves distally, the latches 56 grip the removal member 46, at which point the user may separate the removal device 50 away from the injection device 42, and the needle 41 will be disengaged from the needle adapter 40. In an exemplary embodiment, an audible feedback (e.g., click sound) is provided when the latches 56 grip the removal member 46 and/or when the retaining member 44 becomes dislodged from the retaining element 45.

In an exemplary embodiment, the depth of the opening 53 may be determined based on a length of the needle 41 which extends proximally of the retaining member 45. For example, to prevent the proximal tip of the needle 41 from being exposed when the needle 41 is being removed from the needle adapter 40, the depth of the opening 53 may be selected so that the proximal tip of the needle 41 does not extend beyond an outer surface of the container 51. In another exemplary embodiment, the opening 53 may be enclosed by a cover, valve or septum (not shown). Thus, when the needle 41 is removed from the needle adapter 40, the proximal tip of the needle 41 is shielded by a cover. Increasing the depth of the opening 53 and/or utilizing the cover may prevent needle-stick injuries during removal of the needle 41.

It will be apparent to those skilled in the art that various modifications may be made in the present invention, without departing from the spirit or scope of the invention. Thus, it is intended that the present invention covers the modifications and variations of this invention provided they come within the scope of the appended claims and their equivalents.

## Claims

1. Needle adapter (2) for coupling a needle (3) to an injection device (4), the needle adapter (2) comprising a proximal portion for receiving a distal end of the injection device (4), and a distal portion having an opening for receiving the needle (3).

2. Needle adapter (2) according to claim 1,
**characterized in that** the proximal portion has a screw thread on an inner surface..

3. Needle adapter (2) according to any of the preceding claims,
**characterized in that** the needle adapter (2) includes a longitudinal needle guide (11) formed along a central axis of the needle adapter (2) for receiving the needle (3).

4. Needle adapter (2) according to claim 3,
**characterized in that** the needle adapter (2) includes a retaining element (12) formed along a portion of the needle guide (11) for receiving a retaining member (13) formed on the needle (3).

5. Needle adapter (2) according to claim 4,
**characterized in that** a diameter of the retaining element (12) is larger than a diameter of the needle guide (11).

6. Needle adapter (2) according to claim 4,
**characterized in that** a proximal end of the retaining element (12) prevents proximal displacement of the retaining member (13) in the needle guide (11) beyond the retaining element (12).

7. Needle adapter (2) according to claim 4,
**characterized in that** a tactile feedback is provided when the retaining member (12) is received within the retaining element (12).

8. Needle adapter (2) according to claim 4,
**characterized in that** the retaining member (13) is conically shaped, narrowing in a proximal direction.

9. Needle adapter (2) according to claim 8,
**characterized in that** the opening (7) is conically shaped, narrowing in the proximal direction.

10. Needle adapter (2) according to any one of the preceding claims, **characterized in that** at least one radial slot (8) is formed in the distal portion, wherein at least one end of each of the at least one radial slots terminates at the opening (7).

11. Needle adapter (2) according to claim 10,
**characterized in that** the at least one radial slot (8) forms at least one latch (19).

12. Needle adapter (2) according to claim 11,
**characterized in that** the at least one latch (19) prevents distal displacement of the retaining member (13) in the needle guide (11) beyond the retaining element (12).
